# EUROPEAN PATENT APPLICATION

(11) **EP 3 666 759 A1**
(43) Date of publication of application: **17.06.2020**
(21) Application number: 18211222.7
(22) Date of filing: 10.12.2018
(51) Int. Cl.: C07D 213/61, C07D 401/06

(54) **PREPARATION OF 6-HALO-2-(HALOALKYL)-3-ACYLPYRIDINES AND INTERMEDIATES THEREFOR**

(71) Applicant: Bayer Aktiengesellschaft, 51373 Leverkusen (DE)
(72) Inventor: The designation of the inventor has not yet been filed
(74) Representative: BIP Patents

(57) **Abstract**

The present invention relates to a process for preparing compounds of formula (I) 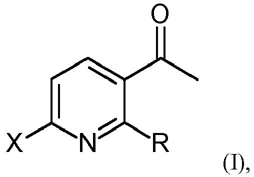 wherein R represents C₁-C₂-alkyl and X represents chlorine or bromine, by reacting a compound of formula (II) 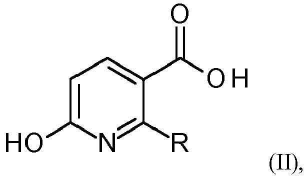 wherein R is defined as in formula (I), in a first step A) with a dehydroxyhalogenation agent selected from COCl₂, (COCl)₂, cyanuric chloride, SOCl₂, SO₂Cl₂, PCl₃, PCl₅, POCl₃, PBr₃, SOBr₂ and SO₂Br₂ to arrive at a compound of formula (III) 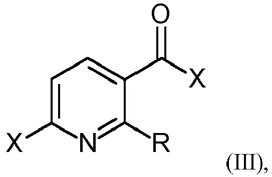 wherein X and R are defined as in formula (I), and the compound of formula (III) is reacted in step B) with a malonate of formula (IV) 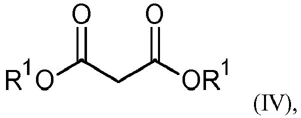 wherein R¹ represents C₁-C₆-alkyl, C₂-C₆-alkenyl, C₂-C₆-alkynyl, C₃-C₈-cycloalkyl or benzyl; in presence of a base and a magnesium compound to arrive at a compound of formula (V) 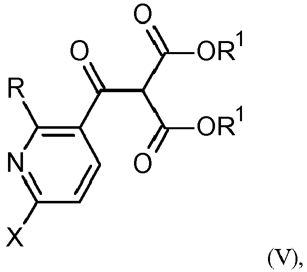 wherein X and R are defined as in formula (I); and R¹ is defined as in formula (IV); and decarboxylating the compound of formula (V). Optionally the resulting compound of formula (I) is further reacted to a triazole derivative of formula (VIII) 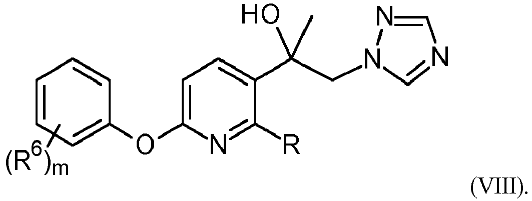 It further relates to the compounds of formula (V).

## Description

The present invention relates to a process for preparing 6-halo-2-(haloalkyl)-3-acylpyridines, and optionally further reacting such pyridines to triazole derivatives showing fungicidal activity. It further relates to key intermediates of said process.

6-halo-2-(haloalkyl)-3-acylpyridines are known to be valuable intermediates in the synthesis of compounds useful in the field of crop protection. WO 2017/029179 A1 discloses fungicidal triazole compounds and several routes to synthesize those. One of said routes is referred to in WO 2017/029179 A1 as process B and comprises synthesis of 6-halo-2-(haloalkyl)-3-acylpyridines which are converted in several steps to triazole derivatives. According to said process B 6-halo-2-(haloalkyl)-3-acylpyridines are formed by converting dihalogenated pyridine derivatives into Grignard compounds and subsequently reacting such Grignard compounds with acyl chlorides to the desired compounds. Although such procedure provides access to the target pyridines it has certain drawbacks. Synthesis of the needed dihalogenated pyridine derivatives is laborious and, hence, the respective pyridine derivatives are rather expensive. Preparation thereof usually involves reacting respective 2-amino-5-halogeno-pyridines with gaseous hydrogen chloride in the presence of dinitrogen trioxide or an organic nitrite. Working with gaseous hydrogen chloride requires special equipment and particular safety precautions. In the not prepublished European patent application 18187203.7 we describe an alternative process that overcomes the disadvantages of the process known from WO 2017/029179 A1. Said alternative process starts from 6-hydroxynicotinic acid derivatives that are reacted with a dehydroxyhalogenation agent and subsequently with an organometallic reagent selected from organolithium compounds and Grignard compounds. This process avoids the need of employing dihalogenated pyridine derivatives and the drawbacks associated therewith, however, still requires handling of organometallic reagents.

Hence, object of the invention is provision of an process for the synthesis of 6-halo-2-(haloalkyl)-3-acylpyridines that avoids the drawbacks of the known methods and provides the desired compounds in excellent yield.

Surprisingly, it has been found that 6-halo-2-(haloalkyl)-3-acylpyridines can be synthesized in high yield starting from certain 6-hydroxynicotinic acid derivatives by reacting those with a dehydroxyhalogenation agent to arrive at respective 2-halonicotinic acid halides and reacting the latter with malonic ester, followed by decarboxylation. This process avoids the need of employing dihalogenated pyridine derivatives as well as organometallic reagents and the drawbacks associated therewith.

Reaction of certain nicotinic acid halogenides with malonic ester and decarboxylation of the resulting compounds is known from Tetrahedron Vol. 48, No. 42, pp. 9233-9236, 1992 and EP 0 341 478 A2. However, said references do not disclose synthesis of any 2-(haloalkyl)-3-acylpyridines, nor deal with the production of fungicides or intermediates thereof.

Accordingly, subject of this invention is a process for preparing a compound of formula (I) 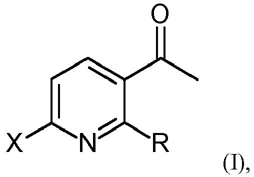 wherein
- R: represents C₁-C₂-haloalkyl; and
- X: represents chlorine or bromine;
characterized in that a compound of formula (II) 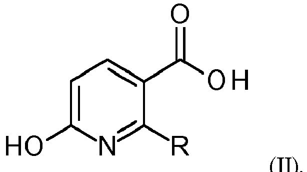 wherein
R is defined as in formula (I);
is reacted in a first step A) with a dehydroxyhalogenation agent selected from COCl₂, (COCl)₂, cyanuric chloride, SOCl₂, SO₂Cl₂, PCl₃, PCl₅, POCl₃, PBr₃, SOBr₂ and SO2Br₂ to arrive at a compound of formula (III) 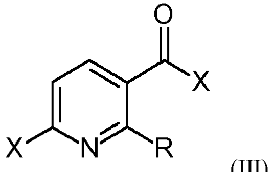
wherein
X and R are defined as in formula (I);
and the compound of formula (III) is reacted in step B) with a malonate of formula (IV) 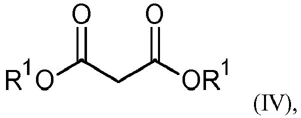
wherein
R¹ represents C₁-C₆-alkyl, C₂-C₆-alkenyl, C₂-C₆-alkynyl, C₃-C₈-cycloalkyl or benzyl;
in presence of a base and a magnesium compound to arrive at a compound of formula (V) 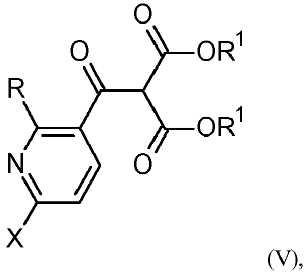
wherein
X and R are defined as in formula (I); and
R¹ is defined as in formula (IV);
and the compound of formula (V) is decarboxylated.

Formula (I) provides a general definition of the pyridines obtainable by the process according to the invention. Preferred radical definitions for formula (I) shown above and below are given below. These definitions apply to compounds of formula (I) and likewise to all educts, intermediates and products bearing the respective radical(s).
- R: preferably represents C₁-haloalkyl.
- R: more preferably represents CF₃, CF₂Cl, CHF₂ or CH₂F.
- R: most preferably represents CF₃.
- X: most preferably represents chlorine.

Formula (II) and (III) provide a general definition of the educt and product of step A) of the process according to the invention. Regarding R and X the definitions given with regard to formula (I) apply mutatis mutandis.

Formula (IV) provides a general definition of the malonate used in step B) of the process according to the invention.
- R¹: preferably represents C₁-C₄-alkyl, C₂-C₆-alkenyl, C₂-C₆-alkynyl, cyclopropyl or benzyl.
- R¹: more preferably represents C₁-C₄-alkyl, C₂-C₆-alkenyl, C₂-C₆-alkynyl or cyclopropyl.
- R¹: more preferably represents C₁-C₄-alkyl or cyclopropyl.
- R¹: more preferably represents methyl, ethyl, n-propyl, isopropyl, n-butyl, isobutyl, tert.-butyl or cyclopropyl.
- R¹: more preferably represents methyl, ethyl, n-propyl, isopropyl, n-butyl, isobutyl or tert.-butyl.
- R¹: more preferably represents methyl or ethyl.
- R¹: most preferably represents methyl.

Formula (V) provides a general definition of an intermediate of step B) of the process according to the invention. Regarding R and X the definitions given with regard to formula (I) apply mutatis mutandis. Regarding R¹ the definitions given with regard to formula (IV) apply mutatis mutandis.

The radical definitions and explanations given above in general terms or stated within preferred ranges can also be combined with one another as desired, i.e. including between the particular ranges and preferred ranges. They apply both to the end products and correspondingly to educts and intermediates. In addition, individual definitions may not apply.

Preference is given to those cases in which each of the radicals have the abovementioned preferred definitions.

Particular preference is given to those cases in which each of the radicals have the abovementioned more and/or most preferred definitions.

Hence, particular preferred is a process for preparing the compound of formula (Ia) 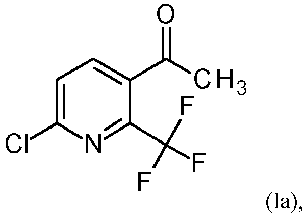 characterized in that the compound of formula (IIa) 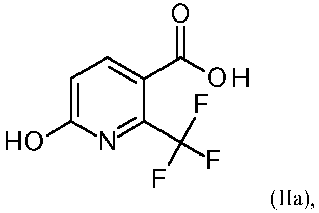 is reacted in a first step A) with a dehydroxyhalogenation agent selected from COCl₂, (COCl)₂, cyanuric chloride, SOCl₂, SO₂Cl₂, PCl₃, PCl₅ or POCl₃ to arrive at a compound of formula (IIIa) 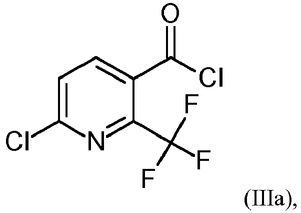 and the compound of formula (IIIa) is reacted in step B) with a malonate of formula (IVa) 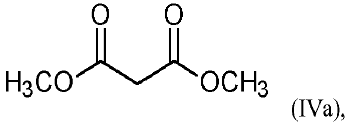 in presence of a base and a magnesium compound to arrive at a compound of formula (Va) 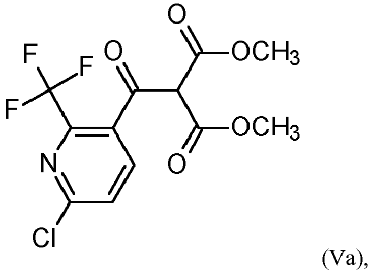 and the compound of formula (Va) is decarboxylated.

In the definitions of the symbols given in the above and below formulae, collective terms were used which are generally representative of the following substituents:
The definition C₁-C₆-alkyl comprises the largest range defined here for an alkyl radical. Specifically, this definition comprises the meanings methyl, ethyl, n-, isopropyl, n-, iso-, sec-, tert-butyl, and also in each case all isomeric pentyls and hexyls, such as methyl, ethyl, propyl, 1-methylethyl, butyl, 1-methylpropyl, 2-methylpropyl, 1,1-dimethylethyl, n-pentyl, 1-methylbutyl, 2-methylbutyl, 3-methylbutyl, 1,2-dimethylpropyl, 1,1-dimethylpropyl, 2,2-dimethylpropyl, 1-ethylpropyl, n-hexyl, 1-methylpentyl, 2-methylpentyl, 3-methylpentyl, 4-methylpentyl, 1,2-dimethylbutyl, 1,3-dimethylbutyl, 2,3-dimethylbutyl, 1,1-dimethylbutyl, 2,2-dimethylbutyl, 3,3-dimethylbutyl, 1,1,2-trimethylpropyl, 1,2,2-trimethylpropyl, 1-ethylbutyl, 2-ethylbutyl, 1-ethyl-3-methylpropyl, in particular propyl, 1-methylethyl, butyl, 1-methylbutyl, 2-methylbutyl, 3-methylbutyl, 1,1-dimethylethyl, 1,2-dimethylbutyl, 1,3-dimethylbutyl, n-pentyl, 1-methylbutyl, 1-ethylpropyl, hexyl, 3-methylpentyl. A preferred range is C₁-C₄-alkyl, such as methyl, ethyl, n-, isopropyl, n-, iso-, sec-, tert-butyl. The definition C₁-C₂-alkyl comprises methyl and ethyl.

The definition halogen comprises fluorine, chlorine, bromine and iodine. Halogen-substitution is generally indicated by the prefix halo, halogen or halogeno.

Halogen-substituted alkyl - e.g. referred to as halogenalkyl, halogenoalkyl or haloalkyl, e.g. C₁-C₄-haloalkyl or C₁-C₂-haloalkyl - represents, for example, C₁-C₄-alkyl or C₁-C₂-alkyl as defined above substituted by one or more halogen substituents which can be the same or different. Preferably C₁-C₄-haloalkyl represents chloromethyl, dichloromethyl, trichloromethyl, fluoromethyl, difluoromethyl, trifluoromethyl, chlorofluoromethyl, dichlorofluoromethyl, chlorodifluoromethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, 1,1-difluoroethyl, pentafluoroethyl, 1-fluoro-1-methylethyl, 2-fluoro-1,1-dimethylethyl, 2-fluoro-1-fluoromethyl-1-methylethyl, 2-fluoro-1,1-di(fluoromethyl)-ethyl, 1-chlorobutyl. Preferably C₁-C₂-haloalkyl represents chloromethyl, dichloromethyl, trichloromethyl, fluoromethyl, difluoromethyl, trifluoromethyl, chlorofluoromethyl, dichlorofluoromethyl, chlorodifluoromethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, 1,1-difluoroethyl, pentafluoroethyl.

The definition C₂-C₆-alkenyl comprises the largest range defined here for an alkenyl radical. Specifically, this definition comprises the meanings ethenyl, n-, isopropenyl, n-, iso-, sec-, tert-butenyl, and also in each case all isomeric pentenyls, hexenyls, 1-methyl-1-propenyl, 1-ethyl-1-butenyl. Halogen-substituted alkenyl - referred to as C₂-C₆-haloalkenyl - represents, for example, C₂-C₆-alkenyl as defined above substituted by one or more halogen substituents which can be the same or different.

The definition C₂-C₆-alkynyl comprises the largest range defined here for an alkynyl radical. Specifically, this definition comprises the meanings ethynyl, n-, isopropynyl, n-, iso-, sec-, tert-butynyl, and also in each case all isomeric pentynyls, hexynyls. Halogen-substituted alkynyl - referred to as C₂-C₆-haloalkynyl - represents, for example, C₂-C₆-alkynyl as defined above substituted by one or more halogen substituents which can be the same or different.

The definition C₃-C₈-cycloalkyl comprises monocyclic saturated hydrocarbyl groups having 3 to 8 carbon ring members, such as cyclopropyl, cyclobutyl, cyclopentyl, cyclohexyl, cycloheptyl and cyclooctyl.

In step A) of the process according to the present invention a compound of formula (II) is reacted with a dehydroxyhalogenation agent, selected from COCl₂, (COCl)₂, cyanuric chloride, SOCl₂, SO₂Cl₂, PCl₃, PCl₅, POCl₃, PBr₃, SOBr₂ and SO₂Br₂.

Preferably, the dehydroxyhalogenation agent is selected from POCl₃, COCl₂ and (COCl)₂, more preferably from POCl₃ and COCl₂, most preferably the dehydroxyhalogenation agent is POCl₃.

Step A) can be conducted in presence or absence of a formamide catalyst. If present, the formamide catalyst is preferably selected from *N,N*-Dimethylformamide, *N,N*-Diethylformamide, *N,N-*Diisopropylformamide, *N,N*-Di-n-butylformamide and mixtures thereof, more preferably the formamide catalyst is *N,N-*Di-n-butylformamide. However, preferably, step A) is conducted in absence of a formamide catalyst.

If present, the amount of formamide catalyst in step A) of the process according to the invention is preferably 0.1 to 10 mole %, more preferably 0.5 to 8 mole %, more preferably 1 to 6 mole %, more preferably 1.5 to 5 mole %, most preferably 2 to 4 mole %, each based on the molar amount of compound of formula (II).

Preferably, step A) is carried out in the presence of an aprotic solvent, preferably selected from aromatic hydrocarbons, chlorinated aromatic hydrocarbons and mixtures thereof, more preferably selected from alkylbenzenes, chlorobenzenes and mixtures thereof, more preferably selected from toluene, xylenes, mesitylene, chlorobenzene and mixtures thereof, more preferred in the presence of toluene and/or xylenes, most preferred in the presence of xylenes.

Preferably, step A) is carried out at a temperature of 20°C to 150°C, preferably 50°C to 145°C.

Preferably, the compound of formula (II) and the dehydroxyhalogenation agent are reacted in a molar ratio of 1 : 1.5 to 1 : 30, preferably 1 : 2 to 1 : 20, more preferred 1 : 2.2 to 1 : 19, even more preferred 1 : 2.5 to 1 : 4.5.

Throughout this specification any reference to a molar amount of a compound or a molar ratio of two reactants is based on the total amount of the compound and reactants, respectively. Therefore, in case a mixture of a particular compound or reactant is present, the molar amount and molar ratio is based on the total amount of all members of this mixture. For example, if a mixture of two or more dehydroxyhalogenation agents is present in step A), the molar ratio is to be calculated based on the total amount of all present dehydroxyhalogenation agents. This applies mutatis mutandis for any reference to an amount by weight or volume and any weight or volume ratios.

Step A) is preferably performed in the presence of a base. These preferably include alkali metal or alkaline earth metal acetates, for example sodium acetate, potassium acetate and calcium acetate, and also basic organic nitrogen compounds, for example trimethylamine, triethylamine, tripropylamine, tributylamine, ethyldiisopropylamine, N,N-dimethylcyclohexylamine, dicyclohexylamine, ethyldicyclohexylamine, N,N-dimethylaniline, N,N-dimethylbenzylamine, pyridine, 2-methyl-, 3-methyl-, 4-methyl-, 2,4-dimethyl-, 2,6-dimethyl-, 3,4-dimethyl- and 3,5-dimethylpyridine, 5-ethyl-2-methylpyridine, 4-dimethylaminopyridine, N-methylpiperidine, 1,4-diazabicyclo[2.2.2]-octane (DABCO), 1,5-diazabicyclo[4.3.0]-non-5-ene (DBN) and 1,8-diazabicyclo[5.4.0]-undec-7-ene (DBU).

Preferably the base is selected from basic organic nitrogen compounds, in particular trimethylamine, triethylamine, tripropylamine, tributylamine, ethyldiisopropylamine, N,N-dimethylcyclohexylamine, dicyclohexylamine, ethyldicyclohexylamine, N,N-dimethylaniline, N,N-dimethylbenzylamine, 1,4-diazabicyclo[2.2.2]-octane (DABCO), 1,5-diazabicyclo[4.3.0]-non-5-ene (DBN), 1,8-diazabicyclo[5.4.0]-undec-7-ene (DBU) and mixtures thereof, more preferably from trimethylamine, triethylamine, tripropylamine, tributylamine, ethyldiisopropylamine, and mixtures thereof. Particular preferred the base is triethylamine.

Preferably, step A) is performed under standard pressure (1 atm). However, it is also possible to work under elevated or reduced pressure. In one particular embodiment step A) is performed under elevated pressure (> 1 atm), preferably at a pressure of 1.2 to 10 bar, more preferred 1.4 to 8 bar, most preferred 1.5 to 5 bar. In this embodiment the preferred and particular preferred dehydroxyhalogenation agent is selected as outlined above.

It is possible to divide step A) into steps A1) and A2), wherein in the first step A1) the compound of formula (II) is reacted with a first dehydroxyhalogenation agent, and the resulting product is reacted in subsequent step A2) with a second dehydroxyhalogenation agent.

The first dehydroxyhalogenation agent used in step A1) is preferably selected from POCl₃, COCl₂, (COCl)₂ and mixtures thereof, more preferably from POCl₃ and COCl₂.

The second dehydroxyhalogenation agent used in step A2) is preferably selected from SOCl₂, SO₂Cl₂ and mixtures thereof, more preferably the second dehydroxyhalogenation agent is SOCl₂.

Preferably, step A1) is carried out in the presence of an aprotic solvent, preferably selected from aromatic hydrocarbons, chlorinated aromatic hydrocarbons and mixtures thereof, more preferably selected from alkylbenzenes, chlorobenzenes and mixtures thereof, more preferably selected from toluene, xylenes, mesitylene, chlorobenzene and mixtures thereof, most preferred in the presence of toluene and/or xylenes.

Preferably, step A1) is carried out at a temperature of 20°C to 150°C, preferably 50°C to 145°C, more preferred 100°C to 145°C.

Preferably, the molar ratio of compound of formula (II) and the dehydroxyhalogenation agent in step A1) is 1 : 1.5 to 1 : 20, preferably 1 : 2 to 1 : 15, more preferred 1 : 2 to 1 : 10, most preferred 1 : 2.2 to 1 : 10.

Preferably, step A1) is carried out in the presence of a base. Description of bases in general and preferred bases given above for step A) apply also for step A1).

Also step A2) is preferably carried out in the presence of an aprotic solvent, preferably selected from aromatic hydrocarbons, chlorinated aromatic hydrocarbons and mixtures thereof, more preferably selected from alkylbenzenes, chlorobenzenes and mixtures thereof, more preferably selected from toluene, xylenes, mesitylene, chlorobenzene and mixtures thereof, most preferred in the presence of toluene and/or xylenes. More preferably, the same kind of solvent that is present in step A1), if any, is also used in step A2).

Preferably, step A2) is carried out at a temperature of 20°C to 150°C, preferably 50°C to 120°C, more preferred 70°C to 110°C.

Preferably, in step A2) the dehydroxyhalogenation agent is present in a molar amount to arrive at a molar ratio of compound of formula (II) to dehydroxyhalogenation agent of 1 : 0.1 to 1 : 15, preferably 1 : 0.15 to 1 : 14, more preferred 1 : 0.15 to 1 : 13, more preferred 1 : 0.2 to 1 : 12, most preferred 1 : 0.2 to 1 : 5.

Preferably, step A2) is carried out without addition of a base.

Preferably, step A2) is performed under standard pressure (1 atm). However, it is also possible to work under elevated or reduced pressure.

Both, step A1) and step A2), can be conducted in presence or absence of a formamide catalyst. Preferably, both steps are conducted in absence of a formamide catalyst.

If present, the amount of formamide catalyst in each of steps A1) and A2) of the process according to the invention is preferably 0.1 to 10 mole %, more preferably 0.5 to 8 mole %, more preferably 1 to 6 mole %, more preferably 1.5 to 5 mole %, most preferably 2 to 4 mole %, each based on the molar amount of compound of formula (II).

The reaction mixture resulting from step A1) can be directly used in step A2) without isolation or purification of the reaction product resulting from step A1).

However, preferably, the reaction mixture resulting from step A1) is worked-up by procedures generally known in the art before using it in step A2). Preferably, after completion of step A1), the reaction mixture is treated with water, an aqueous hydrogen chloride solution, an aqueous sodium chloride solution, an aqueous sodium sulfate solution and/or a saturated aqueous ammonium chloride solution. Preferably, the pH of the resulting mixture is subsequently adjusted to 8-12 by addition of a base, preferably alkali metal hydroxide, more preferred sodium hydroxide, an organic solvent, preferably selected from n-pentane, n-hexane, n-heptane, ethyl acetate and mixtures thereof, is added and the resulting organic and aqueous phases are separated. The aqueous phase is preferably adjusted to pH 1-4, preferably 1-2, by addition of an acid, preferably hydrochloric acid, to initiate precipitation of the desired compound. Preferably, the resulting suspension is cooled to -10 to 10°C, preferably 0 to 5°C to foster further precipitation and crystallization. The resulting precipitate is preferably isolated by filtration and optionally further purified e.g. by washing with water. Finally, the resulting solid may be dried.

If not defined otherwise, any reference to a pH value refers to the value at room temperature (21°C).

The reaction mixture resulting from step A) or A2) may be worked-up by procedures generally known in the art. For example, after completion of step A) or A2), the reaction mixture can be purified by evaporation of volatile components, in particular excess dehydroxyhalogenation agent, preferably under reduced pressure.

If desired to further purify the compound of formula (III), this is possible by known techniques, for example by distillation or treatment with active charcoal and subsequent filtration.

However, a huge advantage of the process according to the invention is that step B) does not require the compound of formula (III) to be of particular purity. Therefore, it is neither necessary to divide step A) into steps A1) and A2), nor to work-up the reaction mixture resulting from step A) or A2), nor to isolate and further purify the compound of formula (III).

Therefore, preferably step A) is performed as a one-step reaction, i.e. not divided into substeps A1) and A2), and the reaction mixture resulting from step A) is directly used in step B) without isolation or purification of the resulting compound of formula (III).

In other words, preferably step A) and step B) are conducted without isolation or purification of the reaction product resulting from step A).

More preferred, after completion of step A) the reaction mixture is quenched with water at a temperature of 0°C to 30°C, preferably 5°C to 25°C, the resulting phases are separated and the organic phase is used in step B).

In step B) the compound of formula (III) is reacted with a malonate of formula (IV) in presence of a base and a magnesium compound to arrive at a compound of formula (V) 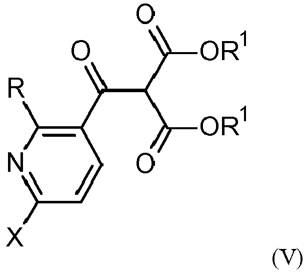 as defined above, and said compound of formula (V) is decarboxylated.

Decarboxylation as used throughout this specification is to be understood as replacing each of the two C(O)OR¹ moieties present in the compound of formula (V) by hydrogen, thereby forming the respective compound of formula (I).

In the following, reacting the compound of formula (III) with a malonate of formula (IV) in presence of a base and a magnesium compound is referred to as step B1) and decarboxylation of the compound of formula (V) as step B2). Hence, step B) comprises steps B1) and B2). Any reference to step B) refers to both steps B1) and B2), whereas any reference to only step B1) or B2) refers to just that step.

Preferably, in step B1) the malonate of formula (IV) is added in a molar amount to arrive at a molar ratio of compound of formula (III) to malonate of formula (IV) of 1 : 0.9 to 1 : 3, preferably 1 : 0.95 to 1 : 2, more preferred 1 : 1 to 1 : 1.5, more preferred 1 : 1 to 1 : 1.2, most preferred 1 : 1 to 1 : 1.1.

Preferably, the base present in step B1) is selected from alkali metal or alkaline earth metal acetates, preferably sodium acetate, potassium acetate and calcium acetate, and basic organic nitrogen compounds, preferably trimethylamine, triethylamine, tripropylamine, tributylamine, ethyldiisopropylamine, N,N-dimethylcyclohexylamine, dicyclohexylamine, ethyldicyclohexylamine, N,N-dimethylaniline, N,N-dimethylbenzylamine, pyridine, 2-methyl-, 3-methyl-, 4-methyl-, 2,4-dimethyl-, 2,6-dimethyl-, 3,4-dimethyl- and 3,5-dimethylpyridine, 5-ethyl-2-methylpyridine, 4-dimethylaminopyridine, N-methylpiperidine, 1,4-diazabicyclo[2.2.2]-octane (DABCO), 1,5-diazabicyclo[4.3.0]-non-5-ene (DBN) and 1,8-diazabicyclo[5.4.0]-undec-7-ene (DBU).

More preferably, the base is selected from basic organic nitrogen compounds, preferably trimethylamine, triethylamine, tripropylamine, tributylamine, ethyldiisopropylamine, N,N-dimethylcyclohexylamine, dicyclohexylamine, ethyldicyclohexylamine, N,N-dimethylaniline, N,N-dimethylbenzylamine, 1,4-diazabicyclo[2.2.2]-octane (DABCO), 1,5-diazabicyclo[4.3.0]-non-5-ene (DBN), 1,8-diazabicyclo[5.4.0]-undec-7-ene (DBU) and mixtures thereof, more preferably from trimethylamine, triethylamine, tripropylamine, tributylamine, ethyldiisopropylamine, and mixtures thereof. Most preferred the base is triethylamine.

Preferably, in step B1) the base is present in a molar amount to arrive at a molar ratio of magnesium compound to base of 1 : 1 to 1 : 5, preferably 1 : 1.5 to 1 : 4, more preferred 1 : 2 to 1 : 3.5.

Preferably, the magnesium compound present in step B1) is selected from MgCl₂, MgBr₂, Mg(OH)₂, Mg(OMe)₂, Mg(OEt)₂, MgO, Mg(NO₃)₂ and mixtures thereof, more preferably from MgCl₂, MgBr₂, MgO and mixtures thereof, more preferably from MgCl₂, MgBr₂ and mixtures thereof. Most preferably, the magnesium compound present in step B1) is MgCl₂.

Preferably, in step B1) the magnesium compound is present in a molar amount to arrive at a molar ratio of compound of formula (III) to magnesium compound of 1 : 0.2 to 1 : 1.5, preferably 1 : 0.3 to 1 : 1.4, more preferred 1 : 0.35 to 1 : 1, more preferred 1 : 0.4 to 1 : 0.8, most preferred 1 : 0.5 to 1 : 0.75.

Preferably, step B1) is carried out in the presence of an aprotic solvent, preferably selected from acetonitrile, ethylacetate, aromatic hydrocarbons, chlorinated aromatic hydrocarbons and mixtures thereof, more preferably selected from acetonitrile, ethylacetate, alkylbenzenes, chlorobenzenes and mixtures thereof, more preferably selected from acetonitrile, ethylacetate, toluene, xylenes, mesitylene, chlorobenzene and mixtures thereof, most preferred selected from toluene, xylenes and mixtures thereof with acetonitrile or ethylacetate.

More preferably, the same kind of solvent that is present in step A), if any, is also used in step B1).

Preferably, step B1) is carried out at a temperature of -20°C to 50°C, preferably 0°C to 40°C, more preferred 10°C to 30°C.

After completion of the reaction, the reaction mixture resulting from step B1) is preferably treated with an aqueous acid, preferably an aqueous hydrochloric acid or aqueous sulfuric acid, more preferably aqueous sulfuric acid, most preferably aqueous sulfuric acid having a concentration of 10 to 30 % by weight. Preferably, the aqueous acid is added in such amount to adjust the pH of the resulting aqueous phase to 1-6, preferably 1-3.

Preferably, resulting organic and aqueous phases are separated and the aqueous phase is extracted with an organic solvent, preferably selected from aromatic hydrocarbons, chlorinated aromatic hydrocarbons and mixtures thereof, more preferably selected from alkylbenzenes, chlorobenzenes and mixtures thereof, more preferably selected from toluene, xylenes, mesitylene, chlorobenzene and mixtures thereof, most preferred from toluene and/or xylenes.

The combined organic phases can be used for the decarboxylation step B2) without any further purification.

If, however, it is desired to isolate the compound of formula (V), this can be achieved by simply removing any organic solvent and volatiles by evaporation in vacuum. If desired, the compounds of formula (V) may be further purified by known techniques, for example chromatography.

In step B2) the compound of formula (V) resulting from step B1) is decarboxylated to arrive at the desired compound of formula (I).

Decarboxylation is preferably performed by heating the compound of formula (V) to at least 50°C, preferably 50°C to 180°C, more preferred 80°C to 170°C, more preferred 85°C to 160°C, most preferred 90°C to 150°C in the presence of water.

Preferably, water is added in an amount of 1 to 30 mol, preferably 2 to 20 mol, more preferred 2 to 10 mol per mol of compound of formula (V).

Since at the elevated temperature used in step B2) some of the water may evaporate from the reaction mixture, it is preferred to add the desired amount of water in several, e.g. 2 to 10, portions or continuously during stirring of the heated reaction mixture.

Optionally, step B2) is performed in the presence of an acid, preferably an aqueous hydrochloric acid or aqueous sulfuric acid, more preferably aqueous sulfuric acid, most preferably aqueous sulfuric acid having a concentration of 10 to 30 % by weight.

Optionally, step B2) is carried out in the presence of an organic solvent, preferably selected from dimethylsulfoxide (DMSO), *N,N*-dimethylformamide (DMF), *N,N*-dimethylacetamide (DMA), acetonitrile, aromatic hydrocarbons, chlorinated aromatic hydrocarbons and mixtures thereof, more preferably selected from alkylbenzenes, chlorobenzenes and mixtures thereof, more preferably selected from toluene, xylenes, mesitylene, chlorobenzene and mixtures thereof, most preferred in the presence of toluene and/or xylenes. More preferably, the same kind of solvent that is present in steps A) and B1), if any, is also used in step B2).

However, preferably step B2) is carried out in the absence of an organic solvent, i.e. organic solvent present in steps A) and B1), if any, is removed from the reaction mixture as outlined above and the decarboxylation is preferably performed in the presence of water and optionally an acid only.

Preferably, step B) is performed under standard pressure (1 atm) or under elevated pressure (> 1 atm), more preferably at least step B2) is performed under elevated pressure, preferably at a pressure of 1.2 to 10 bar, more preferred 1.4 to 8 bar, most preferred 1.5 to 5 bar. Working at elevated pressure allows high rection temperatures, like 110 to 130°C, when the reaction is performed in a mixture of organic solvent and water forming an azeotropic mixture, for example toluene/water or xylene/water.

The compound of formula (I) or solution thereof resulting from step B2) can be directly used in step E) of the process according to the invention outlined below. It is also possible to isolate the compound of formula (I), preferably by evaporation of the organic solvent, preferably under reduced pressure. The process according to the invention yields the compounds of formula (I) in sufficient purity. However, if desired, the compounds of formula (I) may be further purified by known techniques, for example distillation or chromatography. Preferably, the compounds of formula (I) are purified by distillation before use thereof in step E).

The dehydroxyhalogenation agents used in step A) of the process according to the invention as well as the malonates of formula (IV), the bases and magnesium compounds used in step B) are well-known compounds being commercially available or readily available by established synthesis routes.

Compounds of formula (II) are accessible by established synthesis routes starting from compounds disclosed in US 2002/0016345 A1 (page 29, example H7), WO 2004/029026 A1 (page 34, description 26) and WO 2017/162521 A1 (pages 34-35, example PI, step 3) or analog compounds.

However, preferably the compound of formula (II) is prepared by reacting a compound of formula (VI) 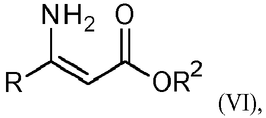 wherein
R is defined as in formula (II); and
R² represents C₁-C₄-alkyl;
in a first step C) with a compound of formula (VII) 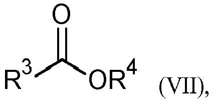
wherein
- R³: represents ethynyl, 1-haloethenyl or 1,2-dihaloethanyl; and
- R⁴: represents C₁-C₄-alkyl;
in the presence of M¹OR⁵, wherein
M¹ represents Li, Na or K; and
R⁵ represents C₁-C₄-alkyl;
and the resulting product is treated in step D) with water in the presence of a base.

R is defined as in formulae (II) and, hence, as in formula (I). The preferred, more preferred and most preferred definitions given with regard to formula (I) apply mutatis mutandis.
- R²: preferably represents methyl, ethyl, n-propyl, isopropyl, n-butyl or tert.-butyl, more preferably methyl, ethyl or n-propyl, most preferably methyl or ethyl.
- R³: preferably represents ethynyl, 1-chloroethen-1-yl or 1,2-dichloroethan-1-yl, more preferably 1,2-dichloroethan-1-yl.
- R⁴: preferably represents methyl, ethyl, n-propyl, isopropyl, n-butyl or tert.-butyl, more preferably methyl, ethyl or n-propyl, most preferably methyl or ethyl.

In step C) compounds of formulae (VI) and (VII) are reacted in the presence of an alcoholate M¹OR⁵.

Preferably, the alkoholate M¹OR⁵ is selected from LiOCH₃, NaOCH₃, KOCH₃, LiOCH₂CH₃, NaOCH₂CH₃, KOCH₂CH₃ and mixtures thereof, more preferably from LiOCH₃, NaOCH₃, KOCH₃, NaOCH₂CH₃ and mixtures thereof. Most preferably, the alkoholate M¹OR⁵ is selected from NaOCH₃ and NaOCH₂CH₃.

Preferably, step C) is carried out in the presence of a C₁-C₄-alcohol or a mixture thereof, preferably methanol, ethanol, isopropanol or a mixture thereof, more preferred methanol, ethanol or a mixture thereof. Most preferably, the alcohol corresponds to the present alcoholate, i.e. the alcohol is methanol in case a methanolate is used, the alcohol is ethanol in case a ethanolate is used and so on.

Preferably, the C₁-C₄-alcohol is present in an amount of 1 to 90 % by weight, based on the total weight of the reaction mixture. More preferably, the C₁-C₄-alcohol is present in an amount of 2 to 80 % by weight, more preferably 5 to 80 % by weight, more preferably 5 to 70 % by weight, more preferably 10 to 70 % by weight, more preferably 20 to 70 % by weight, most preferably 25 to 70 % by weight.

Presence of an additional organic solvent is possible. However, preferably, besides C₁-C₄-alcohol no further organic solvent is present.

Step C) is preferably conducted at a temperature of from 0 to 30 °C, preferably 5 to 30 °C, more preferred 10 to 25 °C and ambient pressure, preferably a pressure of from 0.5 to 2 bar.

Preferably, in step C) the compound of formula (VI) and the compound of formula (VII) are reacted in a molar ratio of 2 : 1 to 1 : 2, preferably 1.5 : 1 to 1 : 1.5, more preferably 1 : 1 to 1 : 1.5.

Preferably, in step C) the molar ratio of compound of formula (VI) and the alkoholate M¹OR⁵ is 1 : 1 to 1 : 10, preferably 1 : 1.5 to 1 : 8, more preferably 1 : 2 to 1 : 6, most preferably 1 : 2 to 1 : 5.

The reaction mixture resulting from step C) can be directly used in step D), i.e. step C) and step D) can be conducted without isolation or purification of the reaction product resulting from step C). Such procedure is preferred.

However, the reaction mixture resulting from step C) can also be worked-up by procedures generally known in the art. If work-up is desired after completion of the reaction, the reaction mixture is preferably filtered to remove any precipitate formed during the reaction. Preferably, the filtrate is concentrated under reduced pressure, optionally washed, preferably with water and/or a saturated aqueous NaCl solution, optionally dried, preferably over magnesium sulfate, and optionally filtered again. The resulting crude product can be used in step D) of the process according to the invention. However, if desired, the crude product may be further purified by known techniques, for example recrystallization or chromatography.

The product resulting from step C) is treated in step D) of the process according to the invention with water in the presence of a base.

The base used in step D) is preferably selected from alkali metal or alkaline earth metal acetates, amides, carbonates, hydrogencarbonates, hydrides, hydroxides or alkoxides, for example sodium acetate, potassium acetate or calcium acetate, lithium amide, sodium amide, potassium amide or calcium amide, sodium carbonate, potassium carbonate or calcium carbonate, sodium hydrogencarbonate, potassium hydrogencarbonate or calcium hydrogencarbonate, lithium hydride, sodium hydride, potassium hydride or calcium hydride, lithium hydroxide, sodium hydroxide, potassium hydroxide or calcium hydroxide, n-butyllithium, sec-butyllithium, tert-butyllithium, lithium diisopropylamide, lithium bis(trimethylsilyl)amide, sodium methoxide, ethoxide, n- or i-propoxide, n-, i-, s- or t-butoxide or potassium methoxide, ethoxide, n- or i-propoxide, n-, i-, s- or t-butoxide; and also basic organic nitrogen compounds, for example trimethylamine, triethylamine, tripropylamine, tributylamine, ethyldiisopropylamine, N,N-dimethylcyclohexylamine, dicyclohexylamine, ethyldicyclohexylamine, N,N-dimethylaniline, N,N-dimethylbenzylamine, pyridine, 2-methyl-, 3-methyl-, 4-methyl-, 2,4-dimethyl-, 2,6-dimethyl-, 3,4-dimethyl- and 3,5-dimethylpyridine, 5-ethyl-2-methylpyridine, 4-dimethylaminopyridine, N-methylpiperidine, 1,4-diazabicyclo[2.2.2]-octane (DABCO), 1,5-diazabicyclo[4.3.0]-non-5-ene (DBN) or 1,8-diazabicyclo[5.4.0]-undec-7-ene (DBU).

Preferably the base is selected from Na₂CO₃, K₂CO₃, Cs₂CO₃, LiOH, NaOH, KOH, NaOMe, KOMe, KOtBu, NaH and mixtures thereof, more preferably from LiOH, NaOH, KOH and mixtures thereof. Particular preferred the base is NaOH.

Preferably, in step D) the base is used in such amount to arrive at a molar ratio of the base and the compound of formula (VII) of 1 : 1 to 10 : 1, preferably 1 : 1 to 8 : 1, more preferably 1.5 : 1 to 5 : 1, most preferably 2 : 1 to 4 : 1.

The amount of water present in step D) can vary in wide limits. However, preferably water is employed in such amount that at least 1 mole water per mole of compound of formula (VII) is present, more preferably at least 2 moles, more preferably at least 5 moles, more preferably at least 10 moles. Particularly preferred, water is used as reaction medium.

It is possible to separately add base and water to the reaction mixture. However, preferably, the base is dissolved in water and the resulting alkaline aqueous solution is added. Additional water may be added, if desired.

Step D) is preferably conducted at a temperature of from 20 to 100 °C, preferably 30 to 90 °C, more preferred 40 to 80 °C, most preferred 50 to 70 °C.

The reaction mixture resulting from step D) can be worked-up by procedures generally known in the art. If work-up is desired after completion of the reaction, the reaction mixture is preferably distilled in vacuum in order to remove the organic solvent. The reaction mixture is then preferably acidified by addition of an acid to a pH of about 2-3 (at room temperature = 21 °C) in order to crystallize the product. Subsequently the solid is preferably filtered, optionally washed, preferably with water, and dried, preferably under vacuum. The process yields the compounds of formula (II) in high purity. However, if desired, the compounds of formula (II) may be further purified by known techniques, for example recrystallisation or chromatography.

The compounds of formula (VII) as well as the alcoholates M¹OR⁵ used in step C) of the process according to the invention are well-known compounds being commercially available or readily obtainable by established synthesis routes.

Same is true regarding the compounds of formula (VI). However, preferably compounds of formula (VI) are prepared by reacting a compound of formula (VIa) 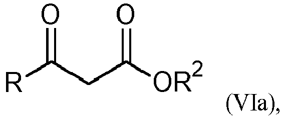 wherein
R and R² are defined as in formula (VI);
with ammonia, an ammonium salt or a mixtures thereof.

The compounds of formula (VIa) are well-known compounds being commercially available or readily obtainable by established synthesis routes.

Preferably, the compound of formula (VIa) is reacted with ammonia, an ammonium salt or a mixture thereof, wherein the ammonium salt is selected from ammonium halogenides and ammonium carboxylates, preferably ammonium fluoride, ammonium chloride, ammonium bromide, ammonium iodide, ammonium formate, ammonium acetate and mixtures thereof, more preferred ammonium chloride, ammonium bromide, ammonium formate, ammonium acetate and mixtures thereof, more preferred ammonium formate, ammonium acetate and mixtures thereof. Most preferred the compound of formula (VIa) is reacted with ammonium acetate.

Preferably, the compound of formula (VIa) and the nitrogen source selected from ammonia, ammonium salt and mixtures thereof is applied in a molar ratio of 1 : 0.8 to 1 : 5, preferably 1 : 1 to 1 : 5, more preferably 1 : 1 to 1 : 4, more preferably 1 : 1 to 1 : 3, most preferably 1 : 1 to 1 : 2.

Preferably, the reaction of compound of formula (VIa) and the nitrogen source selected from ammonia, ammonium salt and mixtures thereof is conducted at a temperature of from 20 to 100 °C, preferably 30 to 90 °C, more preferred 40 to 80 °C, most preferred 50 to 70 °C.

The reaction of compound of formula (VIa) and the nitrogen source selected from ammonia, ammonium salt and mixtures thereof may be conducted in the presence of an organic solvent and/or water. However, preferably this reaction is conducted without addition of any organic solvent and/or water.

The reaction mixture resulting from the reaction of compound of formula (VIa) and the nitrogen source selected from ammonia, ammonium salt and mixtures thereof may be directly used in step C) of the process according to the invention. However, if desired, the reaction mixture can be worked-up by procedures generally known in the art, for example by phase separation and/or distillation in order to purify and/or isolate the resulting compound of formula (VI).

As outlined above, compounds of formula (I) are valuable intermediates in the synthesis of compounds useful in the field of crop protection, in particular the triazole compounds disclosed in WO 2017/029179 A1.

Therefore, the present invention refers furthermore to a process, wherein a compound of formula (I) is synthesized as outlined above and is further reacted to a triazole derivative of formula (VIII) 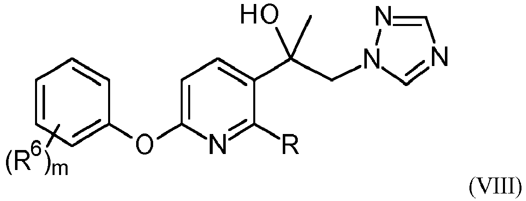 wherein
R is defined as in formula (I);
R⁶ represents halogen, CN, nitro, C₁-C₄-alkyl, C₁-C₄-haloalkyl, C₁-C₄-alkoxy, C₁-C₄-haloalkoxy, C₁-C₄-alkylcarbonyl, hydroxy-substituted C₁-C₄-alkyl or pentafluoro-λ⁶-sulfanyl; and
m is an integer and is 0, 1, 2, 3, 4 or 5;
characterized in that
the reaction of the compound of formula (I) to the triazole derivative of formula (VIII) comprises the following steps:
step E):
   reacting the compound of formula (I) with a phenol derivative of formula (IX) 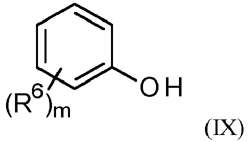 wherein
   R⁶ and m are defined as in formula (VIII);
   in the presence of a base to a compound of formula (X) 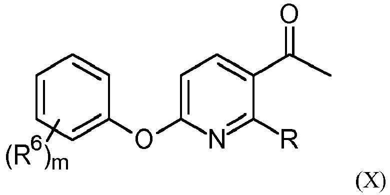
   wherein
   R, R⁶ and m are defined as in formula (VIII);
step F):
   reacting the compound of formula (X) with a trimethylsulfoxonium halide, a trimethylsulfonium halide, trimethylsulfoxonium methylsulfate or trimethylsulfonium methylsulfate to an epoxide of formula (XI) 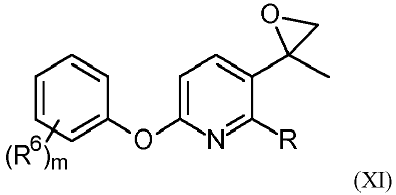 wherein
   R, R⁶ and m are defined as in formula (VIII); and
step G):
   reacting the compound of formula (XI) with 1H-1,2,4-triazole in the presence of a base to the triazole derivative of formula (VIII).

Regarding R the definitions given with regard to formula (I) apply mutatis mutandis.
- R⁶: preferably represents halogen, CN, nitro, C₁-C₄-alkyl, C₁-C₄-haloalkyl, C₁-C₄-alkoxy, C₁-C₄-haloalkoxy or pentafluoro-λ⁶-sulfanyl.
- R⁶: more preferably represents CF₃, OCF₃, Br, Cl or pentafluoro-λ⁶-sulfanyl.
- R⁶: more preferably represents CF₃, OCF₃, Br, Cl or pentafluoro-λ⁶-sulfanyl in the 4-position of the phenyl moiety.
- R⁶: most preferably represents Br or Cl, preferably in the 4-position of the phenyl moiety.
- m: preferably is 1, 2 or 3.
- m: more preferably is 1 or 2.
- m: most preferably is 1.

Preferably, in step E) the compound of formula (I) and phenol derivative of formula (IX) are reacted in a molar ratio of 1 : 1 to 1 : 10, preferably 1 : 1 to 1 : 5, more preferred 1 : 1 to 1 : 3, even more preferred 1 : 1 to 1 : 1.2.

Step E) is carried out in the presence of a base. These preferably include alkali metal or alkaline earth metal acetates, amides, carbonates, hydrogencarbonates, hydrides, hydroxides or alkoxides, for example sodium acetate, potassium acetate or calcium acetate, lithium amide, sodium amide, potassium amide or calcium amide, sodium carbonate, potassium carbonate or calcium carbonate, sodium hydrogencarbonate, potassium hydrogencarbonate or calcium hydrogencarbonate, lithium hydride, sodium hydride, potassium hydride or calcium hydride, lithium hydroxide, sodium hydroxide, potassium hydroxide or calcium hydroxide, n-butyllithium, sec-butyllithium, tert-butyllithium, lithium diisopropylamide, lithium bis(trimethylsilyl)amide, sodium methoxide, ethoxide, n- or i-propoxide, n-, i-, s- or t-butoxide or potassium methoxide, ethoxide, n- or i-propoxide, n-, i-, s- or t-butoxide; and also basic organic nitrogen compounds, for example trimethylamine, triethylamine, tripropylamine, tributylamine, ethyldiisopropylamine, N,N-dimethylcyclohexylamine, dicyclohexylamine, ethyldicyclohexylamine, N,N-dimethylaniline, N,N-dimethylbenzylamine, pyridine, 2-methyl-, 3-methyl-, 4-methyl-, 2,4-dimethyl-, 2,6-dimethyl-, 3,4-dimethyl- and 3,5-dimethylpyridine, 5-ethyl-2-methylpyridine, 4-dimethylaminopyridine, N-methylpiperidine, 1,4-diazabicyclo[2.2.2]-octane (DABCO), 1,5-diazabicyclo[4.3.0]-non-5-ene (DBN) or 1,8-diazabicyclo[5.4.0]-undec-7-ene (DBU).

Preferably the base is selected from Na₂CO₃, K₂CO₃, Cs₂CO₃, KOH, NaOH, NaOMe, KOMe, KOtBu, NaH and mixtures thereof, more preferably from KOH, NaOMe, KOMe, K₂CO₃, Cs₂CO₃ and mixtures thereof. Particular preferred the base is selected from KOH, KOMe, K₂CO₃ and mixtures thereof. Most preferred the base is selected from KOMe and K₂CO₃.

A phenolate nucleophile can be generated *in situ* from the phenol derivative of formula (IX) by use of the abovementioned bases or prepared separately from the phenol derivative of formula (IX) and the base and possibly isolated prior to the reaction. When KOH, NaOMe or KOMe are used as preferred bases to achieve this, the generated water or MeOH is usually distilled off together with all or a portion of any present solvent.

Preferably, step E) is carried out in the presence of a solvent, preferably selected from tetrahydrofuran, methyltetrahydrofuran, in particular 2-methyltetrahydrofuran, diethylether, cyclopropyl methyl ether, *tert*-butyl methyl ether, methyl isobutyl ketone, methyl ethyl ketone, toluene, dimethylformamide (DMF), 1-propanol, 2-propanol, 1-butanol, polyalkylene glycol, in particular polyethylene glycol (PEG), and mixtures thereof. More preferably step E) is carried out in the presence of methyl isobutyl ketone, methyl ethyl ketone, toluene, 1-propanol, 2-propanol, 1-butanol, PEG 400 or mixtures thereof. Most preferably the reaction is carried out in the presence of toluene, 1-propanol, 2-propanol, 1-butanol, PEG 400 or any mixture thereof.

The reaction is completed faster in the presence of a suitable catalyst. Hence, preferably step E) is carried out in the presence of a catalyst, preferably 1,4-diazabicyclo[2.2.2]-octane (DABCO). Preferably, the catalyst is present in an amount of from 1 to 20 mol%, based on the amount of compound of formula (I). Preferably, the reagents used in step E) are mixed at room temperature (21°C). After mixing the reagents, preferably the temperature is increased. Preferably, step E) is carried out at an elevated temperature from 30°C to 150°C, preferably 50°C to 100°C.

The reaction mixture resulting from step E) can be worked-up by procedures generally known in the art. Preferably, after completion of the reaction, the reaction mixture is quenched by addition of water and/or saturated aqueous ammonium chloride solution, the resulting organic and aqueous phases are separated, the aqueous phase is extracted with an organic solvent, preferably toluene, and the combined organic phases are washed, preferably with a saturated aqueous NaCl solution, dried, preferably over magnesium sulfate, and filtered. The resulting solution of the compound of formula (X) or the crude product obtained by evaporation of the organic solvent can be directly used in step F) of the process according to the invention. However, if desired, the compounds of formula (X) may be further purified by known techniques, for example recrystallization or chromatography.

In step F) compounds of formula (X) are converted into epoxides of formula (XI) by reaction with a trimethylsulfoxonium halide, a trimethylsulfonium halide, trimethylsulfoxonium methylsulfate or trimethylsulfonium methylsulfate, preferably trimethylsulfoxonium chloride, trimethylsulfonium chloride, trimethylsulfoxonium methylsulfate or trimethylsulfonium methylsulfate. It is possible to prepare the trimethylsulfoxonium halide, trimethylsulfonium halide, trimethylsulfoxonium methylsulfate or trimethylsulfonium methylsulfate separately before using it in step F). However, it is preferred to prepare said reagents *in situ,* e.g. trimethylsulfonium methylsulfate from a mixture of dimethylsulfide and dimethylsulfate, preferably in the presence of water and optionally a base such as sodium hydroxide or potassium hydroxide.

The trimethylsulfoxonium halide, trimethylsulfonium halide, trimethylsulfoxonium methylsulfate or trimethylsulfonium methylsulfate is preferably used in an amount of 1.1 to 2.5, in particular 1.2 to 2, more preferred 1.3 to 1.6 mole equivalents per 1 mole of compound of formula (X).

Preferably, trimethylsulfonium methylsulfate is used. Particularly preferred an aqueous solution of trimethylsulfonium methylsulfate is used, preferably an aqueous solution containing 38 to 40 wt%, preferably 38 to 39.5 wt%, more preferred 38 to 39.0 wt% of trimethylsulfonium cation. Preferably, said trimethylsulfonium methylsulfate is prepared *in situ,* preferably by mixing dimethylsulfide and water and adding dimethylsulfate to said mixture. Preferably, dimethylsulfide is used in excess, more preferred in an amount resulting in a molar ratio of dimethylsulfate to dimethylsulfide of 1 : 1.5 to 1 : 100, preferably 1 : 2 to 1 : 100, more preferred 1 : 3 to 1 : 90, more preferred 1 : 3.5 to 1 : 80, most preferred 1 : 3.5 to 1 : 5.

Preferably, step F) is carried out at a temperature of -30°C to 50°C, preferably -10°C to 40°C, particularly preferred 20°C to 40°C.

Step F) is preferably conducted in the presence of water, dimethylsulfide, acetonitrile, toluene or a mixture thereof, more preferably in the presence of water, dimethylsulfide or a mixture thereof.

It is preferably carried out in the presence of a base. Preferably the base is selected from Na₂CO₃, K₂CO₃, Cs₂CO₃, NaOH, KOH, KOtBu, NaH and mixtures thereof, more preferably the base is KOH.

The reaction mixture resulting from step F) can be worked-up by procedures generally known in the art. Preferably, after completion of the reaction, the reaction mixture is quenched by addition of water. Preferably, resulting organic and aqueous phases are separated, the aqueous phase is extracted with an organic solvent, preferably tert-butyl methyl ether. The resulting solution of the compound of formula (XI) or the crude product obtained by evaporation of the organic solvent and other volatile components can be directly used in step G) of the process according to the invention. However, if desired, the compounds of formula (XI) may be further purified by known techniques, for example recrystallization or chromatography.

Preferably, in step G) the epoxide of formula (XI) and 1 H-1,2,4-triazole are reacted in a molar ratio of 1 : 1 to 1 : 10, preferably 1 : 1 to 1 : 5, more preferred 1 : 1 to 1 : 3, even more preferred 1 : 1 to 1 : 1.8, most preferred 1 : 1.1 to 1 : 1.2.

Step G) is carried out in the presence of a base. These preferably include alkali metal or alkaline earth metal acetates, amides, carbonates, hydrogencarbonates, hydrides, hydroxides or alkoxides, for example sodium acetate, potassium acetate or calcium acetate, lithium amide, sodium amide, potassium amide or calcium amide, sodium carbonate, potassium carbonate or calcium carbonate, sodium hydrogencarbonate, potassium hydrogencarbonate or calcium hydrogencarbonate, lithium hydride, sodium hydride, potassium hydride or calcium hydride, lithium hydroxide, sodium hydroxide, potassium hydroxide or calcium hydroxide, n-butyllithium, sec-butyllithium, tert-butyllithium, lithium diisopropylamide, lithium bis(trimethylsilyl)amide, sodium methoxide, ethoxide, n- or i-propoxide, n-, i-, s- or t-butoxide or potassium methoxide, ethoxide, n- or i-propoxide, n-, i-, s- or t-butoxide; and also basic organic nitrogen compounds, for example trimethylamine, triethylamine, tripropylamine, tributylamine, ethyldiisopropylamine, N,N-dimethylcyclohexylamine, dicyclohexylamine, ethyldicyclohexylamine, N,N-dimethylaniline, N,N-dimethylbenzylamine, pyridine, 2-methyl-, 3-methyl-, 4-methyl-, 2,4-dimethyl-, 2,6-dimethyl-, 3,4-dimethyl- and 3,5-dimethylpyridine, 5-ethyl-2-methylpyridine, 4-dimethylaminopyridine, N-methylpiperidine, 1,4-diazabicyclo[2.2.2]-octane (DABCO), 1,5-diazabicyclo[4.3.0]-non-5-ene (DBN) or 1,8-diazabicyclo[5.4.0]-undec-7-ene (DBU).

Preferably the base is selected from Na₂CO₃, K₂CO₃, Cs₂CO₃, NaOH, KOH, KOtBu, NaH and mixtures thereof, more preferably from KOH, K₂CO₃, Cs₂CO₃ and mixtures thereof.

In a particular embodiment of this step, a sodium or potassium salt of 1H-1,2,4-triazole is used as base. Said sodium or potassium salt can be prepared by reacting 1H-1,2,4-triazole and a sodium or potassium base, preferably selected from NaOH, NaH and Na-alcoholates or KOH and K-alcoholates, respectively.

Preferably, the base is used in at most stoichiometric amount with respect to the epoxide of formula (XI). More preferred, the epoxide of formula (XI) and the base are reacted in a molar ratio of 1 : 1 to 1 : 0.05, preferably 1 : 0.8 to 1 : 0.05, more preferred 1 : 0.5 to 1 : 0.05, even more preferred 1 : 0.3 to 1 : 0.05.

Preferably, step G) is carried out in the presence of an organic solvent, preferably selected from tetrahydrofuran, methyltetrahydrofuran, in particular 2-methyltetrahydrofuran, diethylether, cyclopropyl methyl ether, *tert*-butyl methyl ether, toluene, C₁-C₆-alcohols, preferably n-butanol, n-propanol, isopropanol, ethanol and methanol, C₁-C₄-alkoxy-C₁-C₆-alcohols, preferably 1-methoxy-propan-2-ol, N-methylpyrrolidinone (NMP), dimethylformamide (DMF), dimethylacetamide (DMAc), dimethylsulphoxide (DMSO), dimethylpropyleneurea (DMPU), dimethylethyleneurea (DMI), tetramethylurea (TMU), N-methylimidazole (NMI), acetonitrile, propionitrile, butyronitrile and mixtures thereof, more preferably in the presence of n-butanol.

Preferably, step G) is carried out at a temperature of 20°C to 150°C, preferably 120°C to 150°C, particularly preferred 100°C to 120°C.

The reaction mixture resulting from step G) can be worked-up by procedures generally known in the art. Preferably, after completion of the reaction, all volatile compounds are evaporated under reduced pressure and the residue is re-dissolved in a suitable organic solvent like dichloromethane or ethyl acetate. Water is added and, if needed, the pH (room temperature) is adjusted to about 6 by introduction of a strong acid like concentrated aqueous hydrochloric acid. The aqueous phase is preferably extracted with a suitable organic solvent like dichloromethane or ethyl acetate. Optionally, the combined organic phases are dried, preferably over magnesium sulfate or sodium sulfate. In case the organic solvent used is dichloromethane drying of the organic phases is not necessary. Preferably, the organic solvent is removed by evaporation and the resulting crude product further purified by known techniques, for example recrystallization or chromatography.

The reaction time of each of the steps of the process according to the invention varies depending on the scale of the reaction and the reaction temperature, but is generally between a few, e.g. 5, minutes and 48 hours.

If not defined otherwise, the process steps according to the invention are generally performed under standard pressure (1 atm). However, it is also possible to work under elevated or reduced pressure.

If not defined otherwise, the amount of any solvent present in any process step according to the invention can vary within wide limits, e.g. from 1 to 99 % by weight, based on the total weight of the respective reaction mixture. Preferrably, the amount of any solvent present in any process step according to the invention is from 1 to 95 % by weight, more preferably from 2 to 90 % by weight, more preferably from 3 to 85 % by weight, more preferably from 4 to 85 % by weight, more preferably from 5 to 80 % by weight, more preferably from 10 to 80 % by weight, more preferably from 15 to 70 % by weight, more preferably from 20 to 70 % by weight, each based on the total weight of the respective reaction mixture.

In step G) an epoxide of formula (XI) is reacted with 1H-1,2,4-triazole to yield the desired triazole compound of formula (VIII). This reaction is sufficiently regioselective, however, yields besides the desired product also minor amounts of the symmetric isomer of formula (VIIIa) 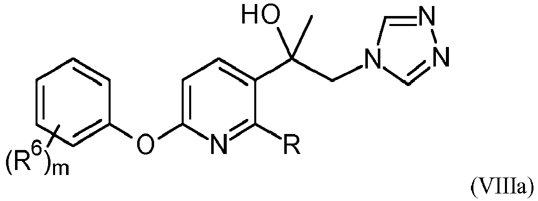 wherein R, R⁶ and m are defined as in formula (VIII).

In case R is CF₃, R⁶ is 4-Br and m is 1, besides the desired compound (VIII-1) 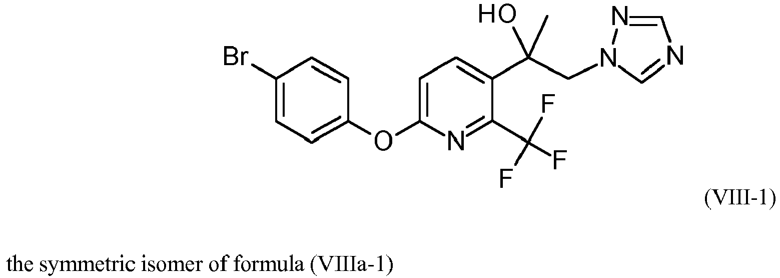 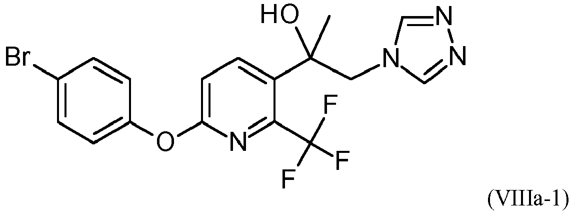 is formed.

If desired, the symmetric isomer of formula (VIIIa-1) can be isolated by conventional means, for example chromatography. By recrystallization at least part of the symmetric isomer of formula (VIIIa-1) can be depleted.

Analytical data of the symmetric isomer of formula (VIIIa-1) are as follows:
¹H-NMR(400.2 MHz, d₆-DMSO):
δ = 8.3129 (9.4); 8.1392 (3.2); 8.1172 (3.5); 7.6459 (0.8); 7.6376 (8.0); 7.6324 (2.7); 7.6207 (2.6); 7.6154 (8.9); 7.6073 (0.9); 7.3144 (4.3); 7.2925 (4.2); 7.2134 (0.9); 7.2052 (8.9); 7.2000 (2.8); 7.1883 (2.5); 7.1830 (8.1); 7.1749 (0.8); 5.9192 (7.8); 5.7566 (5.7); 4.3299 (8.2); 3.3356 (145.7); 2.6765 (0.6); 2.6720 (0.8); 2.6673 (0.6); 2.5251 (2.8); 2.5116 (53.4); 2.5074 (104.4); 2.5030 (135.2); 2.4985 (100.4); 2.4945 (51.4); 2.3345 (0.6); 2.3298 (0.8); 2.3251 (0.6); 1.4701 (16.0); -0.0002 (4.4).

The ¹H-NMR data above are given in form of a ¹H-NMR-peak list. To each signal peak are listed the δ-value in ppm and the signal intensity in round brackets. Between the δ-value - signal intensity pairs are semicolons as delimiters.

The peak list has therefore the form:
δ ₁ (intensity₁); δ₂ (intense₂);........; δᵢ (intensityᵢ);......; δₙ (intensityₙ)

Intensity of sharp signals correlates with the height of the signals in a printed example of a NMR spectrum in cm and shows the real relations of signal intensities. From broad signals several peaks or the middle of the signal and their relative intensity in comparison to the most intensive signal in the spectrum can be shown.

For calibrating chemical shift for ¹H spectra, we use tetramethylsilane and/or the chemical shift of the solvent used, especially in the case of spectra measured in DMSO. Therefore in NMR peak lists, tetramethylsilane peak can occur but not necessarily.

The ¹H-NMR peak lists are similar to classical ¹H-NMR prints and contains therefore usually all peaks, which are listed at classical NMR-interpretation.

Additionally they can show like classical ¹H-NMR prints signals of solvents, stereoisomers of the target compounds and/or peaks of impurities.

To show compound signals in the delta-range of solvents and/or water the usual peaks of solvents, for example peaks of DMSO in DMSO-D₆ and the peak of water are shown in our ¹H-NMR peak lists and have usually on average a high intensity .

The peaks of stereoisomers of the target compounds and/or peaks of impurities have usually on average a lower intensity than the peaks of target compounds (for example with a purity >90%).

Such stereoisomers and/or impurities can be typical for the specific preparation process. Therefore their peaks can help to recognize the reproduction of our preparation process via "side-products-fingerprints". An expert, who calculates the peaks of the target compounds with known methods (MestreC, ACD-simulation, but also with empirically evaluated expectation values) can isolate the peaks of the target compounds as needed optionally using additional intensity filters. This isolation would be similar to relevant peak picking at classical ¹H-NMR interpretation.

Further details of NMR-data description with peak lists you find in the publication "Citation of NMR Peaklist Data within Patent Applications" of the Research Disclosure Database Number 564025.
LogP : 2.47
LogP value is determined by measurement of LC-UV, in an acidic range, with 0.1% formic acid in water and acetonitrile as eluent (linear gradient from 10% acetonitrile to 95% acetonitrile).

In case R is CF₃, R⁶ is 4-Cl and m is 1, the symmetric isomer of formula (VIIIa-2) 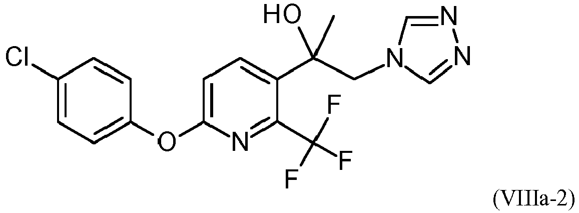 is formed besides the desired compound (VIII-2).

Isolation and depletion of the symmetric isomer of formula (VIIIa-2) can be achieved as outlined above for the symmetric isomer of formula (VIIIa-1).

As mentioned, it is possible to separate the isomers by procedures generally known in the art. Moreover, it is also possible to increase the amount of desired compound of formula (VIII) starting from a mixture comprising the compound of formula (VIII) and the respective symmetric isomer of formula (VIIIa) by crystallization, e.g. in analogy to the process disclosed in WO 2017/102905 A1.

The invention further relates to compounds of formula (V) 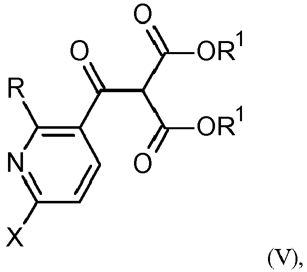 wherein
- R: represents C₁-C₂-haloalkyl;
- R¹: represents C₁-C₆-alkyl, C₂-C₆-alkenyl, C₂-C₆-alkynyl, C₃-C₈-cycloalkyl or benzyl; and
- X: represents chlorine or bromine.

Preferred are compounds of formula (V), wherein R represents C₁-haloalkyl, preferably CF₃, CF₂Cl, CHF₂ or CH₂F, more preferably CF₃.

Preferred are also compounds of formula (V), wherein X represents chlorine.

Preferred are also compounds of formula (V), wherein R¹ represents C₁-C₄-alkyl, C₂-C₆-alkenyl, C₂-C₆-alkynyl, cyclopropyl or benzyl, preferably C₁-C₄-alkyl, C₂-C₆-alkenyl, C₂-C₆-alkynyl or cyclopropyl, more preferably C₁-C₄-alkyl or cyclopropyl, more preferably methyl, ethyl, n-propyl, isopropyl, n-butyl, isobutyl, tert.-butyl or cyclopropyl, more preferably methyl, ethyl, n-propyl, isopropyl, n-butyl, isobutyl or tert.-butyl, more preferably methyl or ethyl, most preferably methyl.

Preference is given to those compounds of formula (V) in which each of the radicals have the abovementioned preferred definitions.

Particular preference is given to those compounds of formula (V) in which each of the radicals have the abovementioned more and/or most preferred definitions.

Hence, particular preferred is the compound of formula (V), wherein
- R: represents CF₃;
- R¹: represents CH₃; and
- X: represents chlorine; i.e. the compound of formula (V-1)

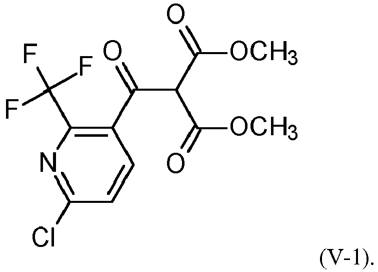

Compounds of formula (V) are obtainable by the process according to the invention as outlined above and are key intermediates in said process.

The invention is illustrated by the examples below. However, the invention is not limited to the examples.

### Examples

### Example 1: Preparation of 6-hydroxy-2-(trifluoromethyl)nicotinic acid from ethyl 3-amino-4,4,4-trifluoro-crotonate and ethyl propiolate (steps C) and D) of the process according to the invention)

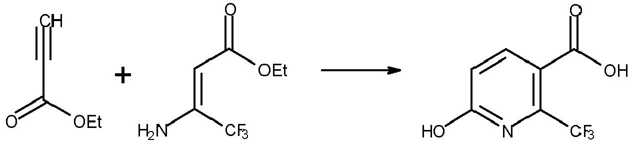

In a 500 mL four-necked flask, equipped with a mechanical stirrer, a bubble counter, a rubber septum and a thermometer, was dissolved under nitrogen 50.0 g (98 % purity, 0.26 mol, 1.0 eq) of ethyl 3-amino-4,4,4-trifluoro-crotonate in 147 mL of sodium ethoxide (21 w% in ethanol) at 22 °C. Afterwards 40.7 mL (99% purity, 0.39 mol, 1.5 eq) of ethyl propiolate was dosed over 2.5 h at 10-18 °C. The dark red solution was stirred for 12 h at this temperature until a HPLC measurement indicated complete conversion of starting materials. Subsequently, 141.5 g of aqueous sodium hydroxide (20 w%) were added slowly to the reaction solution and the resulting mixture was heated to 70 °C internal temperature. At 70 °C some volatiles were distilled off (70 mL) and replaced via addition of deionized water (30 mL). A dark yellow precipitate was obtained. The suspension was cooled to 5-15 °C and was treated with aqueous concentrated hydrogen chloride (37 w%) until pH 2-3 was reached to furnish solid precipitation. The solid was filtered, washed with 2 x 50 mL ice-cold deionized water and dried at 60 °C and 2 mbar vacuum to leave 40.4 g (97% purity, 0.20 mol, 72% yield) of a yellowish solid.
¹H-NMR (400 MHz; DMSO-d6) δ = 8.09 (d, *J* = 8.0 Hz, 1H), 6.96 (d, *J* = 8.0 Hz, 1H).

### Example 2: Preparation of 6-hydroxy-2-(trifluoromethyl)nicotinic acid from ethyl 4,4,4-trifluoro-3-oxo-butanoate and methyl 2,3-dichloropropionate (steps C) and D) of the process according to the invention)

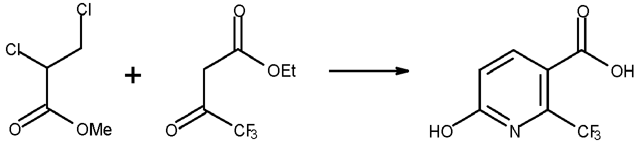

In a 1000 mL four-necked flask, equipped with a mechanical stirrer, a bubble counter, a dosing funnel and a thermometer, was added 200.0 g (98 % purity, 1.06 mol, 1.00 eq) of ethyl 4,4,4-trifluoro-3-oxo-butanoate. After heating to 65 °C internal temperature 143.6 g (1.86 mol, 1.75 eq) of ammonium acetate was added portionwise. After further stirring at 65 °C for 3 h GC monitoring (GC = gas chromatography) revealed complete conversion. The reaction mixture was then cooled to 22 °C and 200 mL of saturated brine were added. The phases were separated and the organic phase was washed with 1 x 200 mL of saturated brine. Consequently, 179.6 g (89% purity, 0.88 mol, 0.83 eq) of ethyl 3-amino-4,4,4-trifluoro-crotonate were obtained as a clear, yellow oil. In a 1000 mL four-necked flask, equipped with a mechanical stirrer, a distillation head with reflux condenser and bubble counter, a dosing funnel and a thermometer the obtained ethyl 3-amino-4,4,4-trifluoro-crotonate was dissolved in 755 mL of sodium methoxide (30 w% in methanol, 2.89 mol, 2.72 eq) at 0 °C. Afterwards 117.6 mL (98% purity, 0.96 mol, 0.91 eq) of methyl 2,3-dichloropropionate was dosed over 1.5 h at 0-10 °C. The resulting yellow suspension was stirred for further 0.5 h at this temperature until a HPLC measurement indicated complete conversion of starting materials. The suspension was heated to 65-70 °C internal temperature and 400 g of methanol were distilled off. Subsequently, 438.8 g of aqueous sodium hydroxide (20 w%, 2.19 mol, 2.06 eq) were dosed slowly to the reaction solution at 70 °C within 1.5 h to result in a yellow suspension. From this mixture another 283 g of methanol were distilled off. Then 150 mL of deionized water were added and the suspension was cooled to 0-10 °C. The suspension was then treated with 280 mL of aqueous concentrated hydrogen chloride (37 w%) until pH 2 was reached to furnish solid precipitation. The solid was filtered, washed with 6 x 250 mL ice-cold deionized water and dried at 40 °C and 10 mbar vacuum to leave 167.6 g (97% purity, 0.79 mol, 74% yield) of a white solid.
¹H-NMR (400 MHz; DMSO-d6) δ = 8.09 (d, *J* = 8.0 Hz, 1H), 6.96 (d, *J* = 8.0 Hz, 1H).

### Example 3: Preparation of 6-chloro-2-(trifluoromethyl)pyridine-3-carbonyl chloride from 6-hydroxy-2-(trifluoromethyl)nicotinic acid (step A) of the process according to the invention)

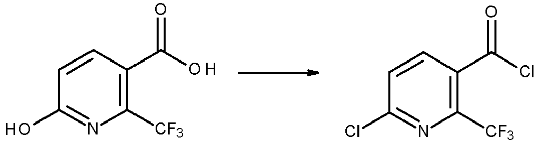

In a four-necked, round-bottomed flask, equipped with a dosing funnel, an inert-gas purge valve, a thermometer and a reflux condenser with a bubble counter, were placed 100.6 g of 2-hydroxy-6-trifluoromethylnicotinic acid and suspended in 400 ml xylenes. To this suspension was added 183.3 g of phosphorous oxychloride at 22°C internal temperature. The mixture was heated to 40°C and 97.14 g of triethylamine (99 % by weight purity) was dosed over 1 hour. The resulting reaction mixture was then further heated to 135-140°C (internal temperature) and stirred at this temperature for 5.0 hours until HPLC (sample quenched with CH₃OH) indicated complete conversion to the intermediate acid chloride. Afterwards the reaction mixture was cooled to 10°C and was dosed onto 500 ml of cold water (10°C) within 2 hours. The two-phasic mixture was stirred at 10°C for 1 hour and the phases were separated. The aqueous phase was extracted once with 200 ml xylenes. The combined organic phase was heated at 50°C in vaccum (20 mbar) and 100 ml xylenes were removed.

To the resulting solution 11.4 g of thionyl chloride were added at 80°C within 1 hour and the solution was then heated further to 105°C internal temperature and stirred for another hour. Volitiles and solvent was removed in vacuum at 50°C (300 mbar - 5 mbar) to give 122 g of the desired product (brown oil, purity: 90,2 % by weight). Yield 94 %.

### Example 4: Preparation of a tautomeric mixture of dimethyl 2-[6-chloro-2-(trifluoromethyl)pyridine-3-carbonyl]propanedioate and methyl (E)-2-[6-chloro-2-(trifluoromethyl)pyridine-3-carbonyl]-3-hydroxy-3-methoxy-prop-2-enoate from 6-chloro-2-(trifluoromethyl)pyridine-3-carbonyl chloride (step B1) of the process according to the invention)

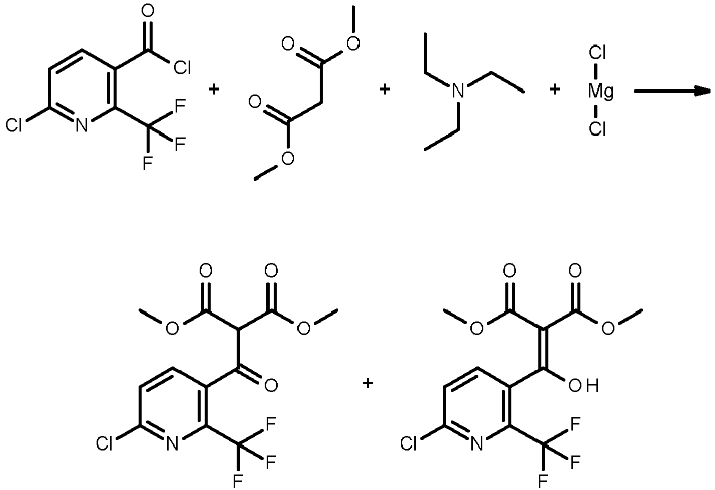

6.65 g (0.07 mol) water free MgCl₂, 13.8 g (0.105 mol) dimethylmalonate and 22 g (0.22 mol) triethylamine in 100 ml xylene were stirred 4 h at room temperature (21°C) to form a white suspension. 24.3 g (0.1 mol) 6-chloro-2-(trifluoromethyl)pyridine-3-carbonyl chloride dissolved in 10 ml of xylene was added to the suspension at 20-22°C within 2-3 h and the mixture was stirred additionally for 1 h. 140 g of 20 % H₂SO₄ was slowly added to the reaction mixture and the resulting mixture stirred for 1 h at room temperature before the phases were separated. The resulting water phase was extracted with 20 ml of xylene and the combined organic extract (110 ml) was used for the decarboxylation step without purification. To isolate the product the xylene phase was evaporated in vacuum to give pale brown oil. Yield: 98 %.

### Analytical characterization:

m/e: 339.

Interpretation ofNMR spectra (¹H-NMR, 600.35 MHz, CD₃CN; ¹³C-NMR, 150.95 MHz, CD₃CN):

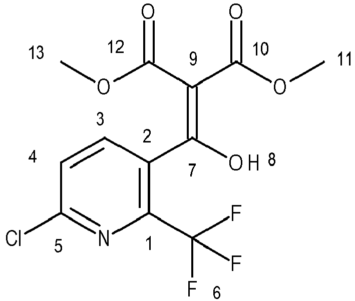

| H/C | δH/ppm | Mult. | rel. No. H | δC/ppm | Mult. | rel. No. C |
|---|---|---|---|---|---|---|
| 1 | - | - | - | 144.7 | S(Q) | 1 |
| 2 | - | - | - | 129.3 | S(Q) | 1 |
| 3 | 7.89 | D | 1 | 142.1 | D | 1 |
| 4 | 7.74 | D | 1 | 128.8 | D | 1 |
| 5 | - | - | - | 152.9 | S | 1 |
| 6 | - | - | - | 121.7 | S(Q) | 1 |
| 7 | - | - | - | 174.4 | s | 1 |
| 8 | 13.66 | S, br | 1 | - | - | - |
| 9 | | | | 103.3 | S | 1 |
| 10 | - | - | - | 165.0 | S | 1 |
| 11 | 3.43 | S | 3 | 52.6 | Q | 1 |
| 12 | - | - | - | 172.0 | S | 1 |
| 13 | 3.89 | S | 3 | 53.8 | S | 1 |

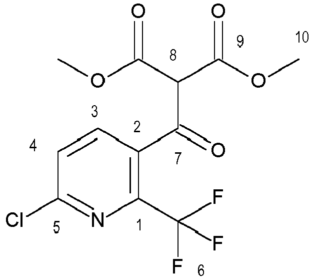

| H/C | δH/ppm | Mult. | rel. No. H | δC/ppm | Mult. | rel. No. C |
|---|---|---|---|---|---|---|
| 1 | - | - | - | 144.9 | S(Q) | 1 |
| 2 | - | - | - | 133.6 | S(Q) | 1 |
| 3 | 8.12 | D | 1 | 140.9 | D | 1 |
| 4 | 7.77 | D | 1 | 128.8 | D | 1 |
| 5 | - | - | - | 153.5 | S | 1 |
| 6 | - | - | - | 121.4 | S(Q) | 1 |
| 7 | - | - | - | 191.5 | S | 1 |
| 8 | 5.30 | S | 1 | 65.0 | D | 1 |
| 9 | - | - | - | 165.4 | S | 2 |
| 10 | 3.75 | D | 6 | 54.1 | Q | 2 |

### Example 5: Preparation of a tautomeric mixture of dimethyl 2-[6-chloro-2-(trifluoromethyl)pyridine-3-carbonyl]propanedioate and methyl (E)-2-[6-chloro-2-(trifluoromethyl)pyridine-3-carbonyl]-3-hydroxy-3-methoxy-prop-2-enoate from 6-chloro-2-(trifluoromethyl)pyridine-3-carbonyl chloride (step B1) of the process according to the invention)

In a four-necked, round-bottomed flask equipped with a syringe pump, a condenser, a mechanical stirrer and a thermometer 13.6 g of MgCl₂ was suspended in 270 ml of xylene. 44.5 g of triethylamine and 29.6 g of dimethylmalonate were added to the mixture subsequently. The suspension was stirred for 4 h at 22°C and finally 100 ml of ethylacetate was added to the reaction mixture. 53.93 g of 6-chloro-2-(trifluoromethyl)pyridine-3-carbonyl chloride (purity 90.2 %) disolved in 30 ml of xylene was added within 1-2 h at 22°C to the suspension and the mixture was stirred for 3 h at room temperature. To the good stirrable yellow suspension (185 ml) H₂SO₄ (10 %) was added at 15°C to achieve pH of 1-2. The precipitate disappeared and a clear two phase mixture formed. The phases were separated and the water phase was extracted with 100 ml of ethylacetate. The combined extract was washed twice with 50 ml of water/brine each and the organic phase was concenrated in vaccum (300-5 mbar at 40-60°C). The oily residue slowly solidified to yield a yellow-brown solid.

Yield: 72 g, purity 94 % by weight (sum of the two components ketone and enol).

### Example 6: Preparation of 1-[6-chloro-2-(trifluoromethyl)-3-pyridyl]ethanone by decarboxylation of the tautomeric mixture prepared in example 4 and 5 (step B2) of the process according to the invention)

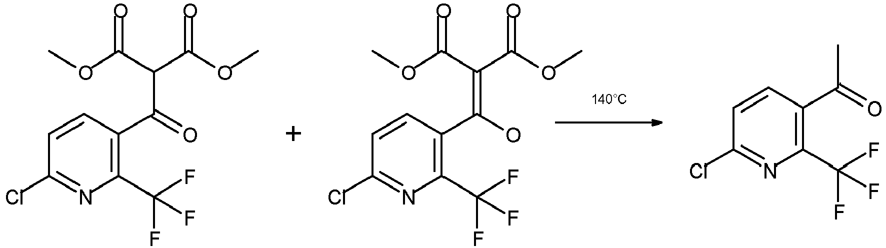

The solution of dimethyl 2-[6-chloro-2-(trifluoromethyl)pyridine-3-carbonyl]propanedioate and methyl (E)-2- [6-chloro-2-(trifluoromethyl)pyridine-3 -carbonyl] -3 -hydroxy-3 -methoxy-prop-2-enoate from example 4 and 5, respectively, was heated to 140°C and 10 ml of water was added to the mixture within 8-10 h using a syringe pump. Low boiling products formed during the reaction were distilled off to keep the temperature inside the flask at 140°C. Thereafter, the solvent was evaporated to yield crude 1-[6-chloro-2-(trifluoromethyl)-3-pyridyl]ethanone. Purity: 88-92 % by weight. Yield: 95 %.

m/e: 223.
¹H NMR (400 MHz; DMSO-d6): δ =8,4, d, (1H); 8,03 d (1H), 2,63 s (3H) ppm.

### Example 7: Preparation of 1-[6-chloro-2-(trifluoromethyl)-3-pyridyl]ethanone by decarboxylation of the mixture prepared in example 4 at elevated pressure (step B2) of the process according to the invention)

The solution of dimethyl 2-[6-chloro-2-(trifluoromethyl)pyridine-3-carbonyl]propanedioate and methyl (E)-2- [6-chloro-2-(trifluoromethyl)pyridine-3 -carbonyl] -3 -hydroxy-3 -methoxy-prop-2-enoate from example 4 and 10 ml water were filled in an autoclave and heated to 125°C for 8-10 h. The autoclave was opened and the obtained solution was concentrated in vacuo (20 mbar) and 50°C to yield crude 1-[6-chloro-2-(trifluoromethyl)-3-pyridyl]ethanone with a purity of 92 % by weight. Yield: 99 %.

### Example 8: Preparation of 2-[6-(4-bromophenoxy)-2-(trifluoromethyl)pyridin-3-yl]-1-(1H-12.4-triazol-1-yl)propan-2-ol from 1-[6-chloro-2-(trifluoromethyl)-3-pyridyl]ethanone (steps E) to G) of the process according to the invention)

### Step E: 1-[6-(4-bromophenoxy)-2-(trifluoromethyl)-3-pyridyl]ethanone

In a 100 mL three-necked roundbottom flask equipped with an overhead-stirrer, dropping funnel and thermometer 7.3 g 4-bromophenole (41.8 mmol, 1.05 equiv (equiv = equivalents)) was dissolved in 20 mL of 2-propanol at room temperature. 3.4 g K₂CO₃ powder (24.3 mmol), 0.61 equiv) and 223 mg 1,4-diazabicyclo(2.2.2)octane (1.99 mmol, 0.05 equiv) were added at room temperature. The suspension was heated to 81 °C inner temperature at which 10.0 g 1-[6-chloro-2-(trifluoromethyl)-3-pyridyl]ethanone (39.8 mmol, 1.0 equiv) was dosed over 2 h. The mixture was further stirred at this temperature for 11 h. HPLC analysis showed >99% conversion. The mixture was cooled to room temperature and was kept stirring at this temperature overnight. Water was then added to precipitated the desired product. The solid was filtered off, washed with water and dried by suction to obtain 14.4 g of the product as light-beige crystals of 97.7 % by weight purity (98.3 % yield).

### Step F: 6-(4-bromophenoxy)-3-(2-methyloxiran-2-yl)-2-(trifluoromethyl)pyridine

In a 2 L reactor equipped with an overhead-stirrer, dropping funnel, thermometer and several bleach containing scrubbers 128.6 g dimethylsulfide (2.05 mol, 6.0 equiv) and 6.2 mL water were introduced and heated to 32 °C inner temperature. 65.3 g dimethylsulfate (0.51 mol, 1.5 equiv) was added within 30 minutes. The temperature rose to 39.5 °C and the mixture turned cloudy. The mixture was stirred at 37.5 °C inner temperature for 4 h. The mixture was cooled to room temperature and 124.0 g 1-[6-(4-bromophenoxy)-2-(trifluoromethyl)-3-pyridyl]ethanone (0.34 mol, 1.0 equiv) was added at room temperature. The mixture was stirred 20 minutes before being heated to an inner temperature of 38-40 °C at which 57.5 g KOH pellets (1.03 mol, 3.0 equiv) were added. The mixture was further stirred at 42 °C for 4 h and afterwards cooled to room temperature and kept overnight. HPLC analysis showed full conversion. The mixture was diluted with 400 mL water and 500 mL methyl *tert*-butyl ether (MTBE), filtered through a plug of Celite®, the phases were separated and the organic phase was dried over Na₂SO₄ and evaporated. The crude product was redissolved in 450 mL MTBE and the remaining volatiles were evaporated to obtain 121 g of a redbrown oil of 94.3 a/a-% HPLC purity that crystallizes upon standing (89.3 % yield).

¹H NMR (600 MHz; CD₃CN): δ =8.09, d, (1H); 7.60, d, (2H); 7.22, d, (1H); 7.14, d, (2H); 3.05, d, (1H); 2.90, d, (1H); 1.62 s (3H) ppm.

### Step G: 2-[6-(4-bromophenoxy)-2-(trifluoromethyl)pyridin-3-yl]-1-(1H-1,2,4-triazol-1-yl)propan-2-ol

225 g of 6-(4-bromophenoxy)-3-(2-methyloxiran-2-yl)-2-(trifluoromethyl)pyridine (0.575 mol, 1 equiv) was added to a mixture of 608 mL of n-butanol and 68 mL of PEG400. 60.6 g of 1H-1,2,4-triazole (0.862 mol, 1.5 equiv) and 15.2 g of K₂CO₃ powder (0.23 mol, 0.4 equiv) were added and the mixture was heated to 100 °C for 13 h. The solvent was removed under reduced pressure until a viscous light-brown oil remained. 3 L of ethyl acetate were added together with 1.5 L of water and 18 mL of concentrated aqueous hydrochloric acid. The phases were separated and the organic phase was washed with water. The organic phase was dried over Na₂SO₄ and the solvent was removed under reduced pressure. The remaining oily residue crystallized quickly and was triturated 3 times with cyclohexane. The wet product was filtered and dried, yielding 240 g of beige crystals of 90 a/a-% HPLC purity (84.7% yield). The crystals were dissolved in 760 mL of 2-propanol at 82 °C. 1170 mL of water were added and after stirring at 80 °C for 5 minutes, the mixture was let cool down to room temperature overnight. The suspension was further cooled down to 10-15 °C, filtered and washed with 800 mL of water. The product was dried under vacuum at 50 °C to obtain 202 g of light-beige crystals of 97.9 a/a-% HPLC purity (77.6% yield).

## Claims

1. Process for preparing a compound of formula (I) 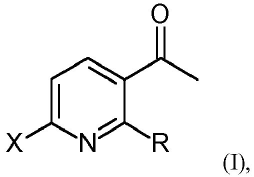 wherein
R represents C₁-C₂-haloalkyl; and
X represents chlorine or bromine;
**characterized in that** a compound of formula (II) 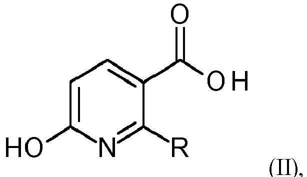 wherein
R is defined as in formula (I);
is reacted in a first step A) with a dehydroxyhalogenation agent selected from COCl₂, (COCl)₂, cyanuric chloride, SOCl₂, SO₂Cl₂, PCl₃, PCl₅, POCl₃, PBr₃, SOBr₂ and SO₂Br₂, to arrive at a compound of formula (III) 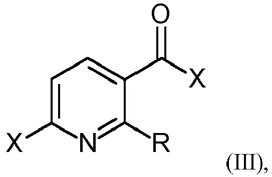
wherein
X and R are defined as in formula (I);
and the compound of formula (III) is reacted in step B) with a malonate of formula (IV) 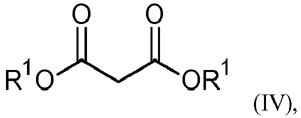
wherein
R¹ represents C₁-C₆-alkyl, C₂-C₆-alkenyl, C₂-C₆-alkynyl, C₃-C₈-cycloalkyl or benzyl;
in presence of a base and a magnesium compound to arrive at a compound of formula (V) 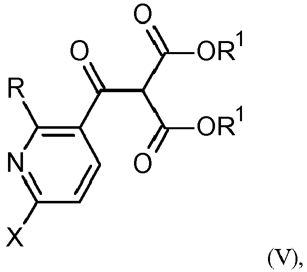
wherein
X and R are defined as in formula (I); and
R¹ is defined as in formula (IV);
and the compound of formula (V) is decarboxylated.

2. Process according to claim 1, wherein R represents CF₃.

3. Process according to at least one of claims 1 to 2, wherein X represents chlorine.

4. Process according to at least one of claims 1 to 3, wherein R¹ represents methyl, ethyl, n-propyl, isopropyl, n-butyl, isobutyl or tert.-butyl.

5. Process according to at least one of claims 1 to 4, wherein the dehydroxyhalogenation agent is selected from COCl₂ and POCl₃.

6. Process according to at least one of claims 1 to 5, wherein step A) is conducted in the presence of an organic solvent selected from toluene, xylene and mixtures thereof and/or in the presence of a base selected from trimethylamine, triethylamine, tripropylamine, tributylamine, ethyldiisopropylamine, N,N-dimethylcyclohexylamine, dicyclohexylamine, ethyldicyclohexylamine, N,N-dimethylaniline, N,N-dimethylbenzylamine, 1,4-diazabicyclo[2.2.2]-octane (DABCO), 1,5-diazabicyclo[4.3.0]-non-5-ene (DBN), 1,8-diazabicyclo[5.4.0]-undec-7-ene (DBU) and mixtures thereof.

7. Process according to at least one of claims 1 to 6, wherein step A) and step B) are conducted without isolation or purification of the reaction product resulting from step A).

8. Process according to at least one of claims 1 to 7, wherein the base present in step B) is selected from trimethylamine, triethylamine, tripropylamine, tributylamine, ethyldiisopropylamine, N,N-dimethylcyclohexylamine, dicyclohexylamine, ethyldicyclohexylamine, N,N-dimethylaniline, N,N-dimethylbenzylamine, 1,4-diazabicyclo[2.2.2]-octane (DABCO), 1,5-diazabicyclo[4.3.0]-non-5-ene (DBN), 1,8-diazabicyclo[5.4.0]-undec-7-ene (DBU) and mixtures thereof.

9. Process according to at least one of claims 1 to 7, wherein the base present in step B) is, trimethylamine.

10. Process according to at least one of claims 1 to 9, wherein the magnesium compound present in step B) is selected from MgCl₂, MgBr₂, Mg(OH)₂, Mg(OMe)₂, Mg(OEt)₂, MgO, Mg(NO₃)₂ and mixtures thereof.

11. Process according to at least one of claims 1 to 9, wherein the magnesium compound present in step B) is MgCl₂.

12. Process according to at least one of claims 1 to 11, wherein in step B) the molar ratio of compound of formula (III) and magnesium compound is 1 : 0.4 to 1 : 0.8.

13. Process according to at least one of claims 1 to 12, wherein decarboxylation of the compound of formula (V) is performed by heating the compound in the presence of water to 50 to 180°C.

14. Process according to at least one of claims 1 to 13, wherein step B) is conducted in the presence of an organic solvent selected from toluene, xylene and mixtures thereof with acetonitrile or ethylacetate.

15. Process according to at least one of claims 1 to 14, wherein the compound of formula (II) is prepared by reacting a compound of formula (VI) 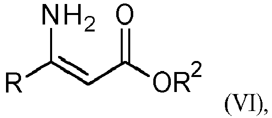 wherein
R is defined as in formula (II); and
R² represents C₁-C₄-alkyl;
in a first step C) with a compound of formula (VII) 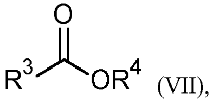
wherein
R³ represents ethynyl, 1-haloethenyl or 1,2-dihaloethanyl; and
R⁴ represents C₁-C₄-alkyl;
in the presence of M¹OR⁵, wherein
M¹ represents Li, Na or K; and
R⁵ represents C₁-C₄-alkyl;
and the resulting product is treated in step D) with water in the presence of a base.

16. Process according to at least one of claims 1 to 15, wherein compound of formula (I) is further reacted to a triazole derivative of formula (VIII) 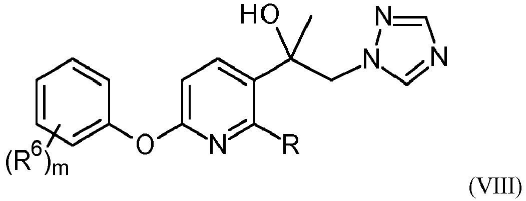 wherein
R is defined as in formula (I);
R⁶ represents halogen, CN, nitro, C₁-C₄-alkyl, C₁-C₄-haloalkyl, C₁-C₄-alkoxy, C₁-C₄-haloalkoxy, C₁-C₄-alkylcarbonyl, hydroxy-substituted C₁-C₄-alkyl or pentafluoro-λ⁶-sulfanyl; and
m is an integer and is 0, 1, 2, 3, 4 or 5;
**characterized in that**
the reaction of the compound of formula (I) to the triazole derivative of formula (VIII) comprises the following steps :
step E):
reacting the compound of formula (I) with a phenol derivative of formula (IX) 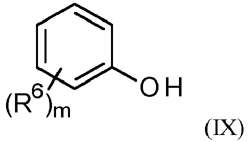 wherein
R⁶ and m are defined as in formula (VIII);
in the presence of a base to a compound of formula (X) 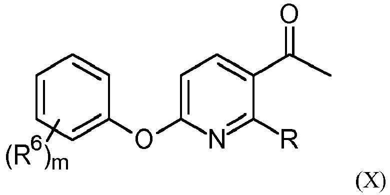
wherein
R, R⁶ and m are defined as in formula (VIII);
step F):
reacting the compound of formula (X) with a trimethylsulfoxonium halide, a trimethylsulfonium halide, trimethylsulfoxonium methylsulfate or trimethylsulfonium methylsulfate to an epoxide of formula (XI) 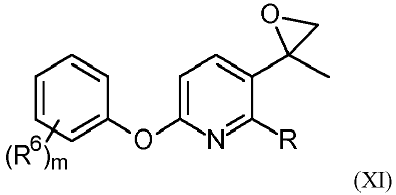 wherein
R, R⁶ and m are defined as in formula (VIII); and
step G):
reacting the compound of formula (XI) with 1H-1,2,4-triazole in the presence of a base to the triazole derivative of formula (VIII).
Compound of formula (V) 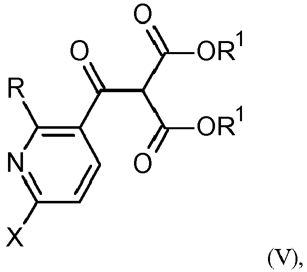 wherein
R represents C₁-C₂-haloalkyl;
R¹ represents C₁-C₆-alkyl, C₂-C₆-alkenyl, C₂-C₆-alkynyl, C₃-C₈-cycloalkyl or benzyl; and
X represents chlorine or bromine.
Compound according to claim 17, wherein
R represents CF₃;
R¹ represents CH₃; and
X represents chlorine.
